# EUROPEAN PATENT SPECIFICATION

(11) **EP 1 345 501 B1**
(45) Date of publication and mention of the grant of the patent: **17.01.2007**
(21) Application number: 01272624.6
(22) Date of filing: 10.10.2001
(51) Int. Cl.: A23L 1/30, A23L 1/221, A61K 45/06

(54) **NUTRITIONAL AND THERAPEUTICAL PREPARATIONS HAVING ANTIOXIDANT ACTIVITY**
NAHRUNGS- UND THERAPIEPRODUKTE MIT ANTIOXIDATIONSWIRKUNG
PREPARATIONS NUTRITIONNELLES ET THERAPEUTIQUES PRESENTANT UNE ACTIVITE ANTIOXYDANTE

(30) Priority: 29.12.2000 IT MI20002854
(43) Date of publication of application: 24.09.2003
(73) Proprietor: Hunza di Pistolesi Elvira E C. S.a.S., 20148 Milan (IT)
(72) Inventor: PISTOLESI, ELvira, I-20148 Milano (IT)
(74) Representative: Minoja, Fabrizio
(86) International application number: PCT/EP2001/011736
(87) International publication number: WO 2002/052955

(56) References cited:
- EP-A- 0 180 786
- WO-A-00/43036
- WO-A-93/00015
- WO-A-99/22751
- GB-A- 350 684
- DATABASE WPI Section Ch, Week 198629 Derwent Publications Ltd., London, GB; Class B05, AN 1986-186011 XP002190617 & JP 61 118318 A (PALA KASEI KOGYO KK), 5 June 1986 (1986-06-05)
- DATABASE WPI Section Ch, Week 198819 Derwent Publications Ltd., London, GB; Class B05, AN 1988-129547 XP002190811 & JP 63 072654 A (SANMARU KAGAKU KK), 2 April 1988 (1988-04-02)

## Description

The present invention relates to pharmaceutical and dietary compositions, as well as to functional foodstuffs useful as coadjuvants for both preventing and treating aging processes and related conditions: atherosclerosis, hypertension, diabetes, tumors, obesity and overweight, hypertriglyceridemia, hypercholesterolemia, aging of the skin, alopecia, panniculitis (cellulite), osteoporosis, cerebral aging (Alzheimer, Parkinson, senile dementia, etc.) and loss of memory, stress, depression, menopausal syndromes, benign prostate hypertrophy, and the like. An anti-ageing pill comprising omega-3 fatty acids extracted from fish, natural vitamin E, lipoic acid, a mix of monoterpenes and polycosanols is disclosed by A. Maschitto (SALVE, Sept. 2000 pages 25-26)

The composition according to the present invention comprises :
a) a lipidic mixture rich in polyunsaturated fatty acids, preferably docosahexaenoic acid (DHA), eicosapentaenoic acid (EPA), conjugated linoleic acids (CLA) and γ-linolenic acid, and antioxidant vitamins, in combination with :
b) one or more terpenes, selected from monoterpenes and/or sesquiterpenes, triterpenes, lactonic terpenes, but preferably monoterpenes or sesquiterpenes,
c) 1-piperoylpiperidine (in pure form and/or purified extracts or fractions containing it enriched in black pepper) and/or capsaicin and analogues thereof, preferably 1-piperoylpiperidine, and optionally
d) one or more polycosanols and/or polycosanolic acids.

The lipidic mixture can contain the polyunsaturated fatty acids in the form of tri-, di- and monoglycerids, phospholipid esters, free acids or alkali or alkaline-earth metal salts, salts with amino acids (arginine, lysine, methionine, cysteine, and the like) or salts with nitrogen bases such as ethanolamine, mono- and dimethylethanolamine, choline, and the like, or as ethyl esters. Preferred sources of these unsaturated acids are oils from sea animals (from cod, mackerel, tuna, seal, etc.) and/or oils from different types of seeds (maize, soy-bean, sunflower, colza, wheat germ, borage, "evening primrose", red currant, linseed, perilla, etc.), olive, seaweed, pepper, mushrooms (for example Mucor javanicus) oils, lecithins and phospholipids of different nature, etc. with various purity degrees.

The antioxidant vitamins of the lipide mixture can be selected from one or more of the following compounds:
vitamin E, lipoic acid, vitamin C as such and/or as acyl derivative, vitamin B6 as such and/or as acyl derivative, folic acids, vitamin B12, etc.; water-soluble vitamins (vitamin B6, folic acids, vitamin B12, etc.) are preferably added in the form of complexes with lecithins which promote their dispersion in the lipidic mixture.

Examples of monoterpenes include d-limonene, β-caryophyllene, sabinene, borneol, carveol, carvone, eugenol, eugenol acetate, geraniol, menthol, myrtanol, pinene, β-pinene, thymol, and the like.

Examples of sesquiterpenes include abscisic acid, β-ionone, rodinal, and the like.

Examples of lactonic terpenes include ginkgolides, bilobalides, and the like.

Examples of triterpenes include madecassic acid, asiatic acid, asiaticoside, etc.

Preferred sources of these terpenes are suitably purified fractions of:
A) fruit or vegetable or spices essential oils rich in limonene, thymol, menthol, etc.. and
B) medicinal herbs essential oils (such as Ginkgo biloba, Centella asiatica, etc..) rich in lactonic terpenes and triterpenes.

1-Piperoylpiperidine, of formula: 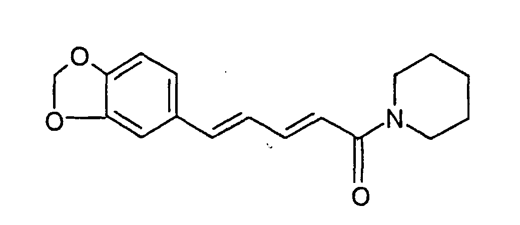 can be present as pure compound or it can be contained in black pepper (piper nigrum) extracts.

Examples of vegetable extracts containing 1-piperoylpiperidine are extracts in apolar solvents of black pepper fruits which, after evaporation of the solvent, may either be used crude or be further purified by column chromatography or other conventional techniques to obtain highly pure fractions of 1-piperoylpiperidine (above 95%).

Capsaicin and its analogues can be present as pure compounds or contained in extracts of red pepper (capsicum annuum L.), chili, paprika, etc..

Examples of polycosanols (long chain aliphatic alcohols: C ≥ 24) include octacosanol, triacosanol, tetracosanol and hexacosanol. Preferred sources of these polycosanols are suitably purified wax fractions of maize, wheat, rice, citrus fruits, sugar cane, and the like.

Examples of polycosanolic acids (saturated long chain fatty acids: C≥24, 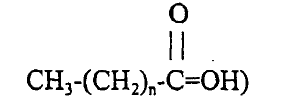 are octacosanolic acid (montanic acid), triacosanolic, tetracosanolic, hexacosanolic acids, and the like. Preferred sources of these polycosanolic acids are suitably purified wax fractions of maize, wheat, rice, citrus fruits, sugar cane, sea animals fats, and the like. Alternatively, these fatty polycosanolic acids can easily be obtained by chemical oxidation of the alcohol group of the corresponding polycosanols according to conventional oxidation procedures.

The preparation of octacosanolic acid from the corresponding octacosanol (or a mixture of polycosanols containing it) is reported in the following by way of example.

Octacosanol was dissolved in 200 ml of acetic acid, treated with 75 g of chromic acid in acetic acid, and left for 2 hr at 0°C and 1 hr at room temperature. Dilute hydrochloric acid and benzene were added to the mixture and the extract was evaporated under reduced pressure. The residual oil was partitioned between benzene and a solution of 5% sodium hydroxide in water-methanol 2:1. From the benzene layer, 30 g of neutral material was recovered. An oil that separated from the aqueous layer was acidified with hydrochloric acid and treated with benzene, which was evaporated to give 40 g of octacosanolic acid. The methyl ester of the latter was pure by GLC analysis. The product was distilled at 170°C/0.01 mm. This method is a partial modification of the procedure by Steinberg for the preparation phytanic acid from the corresponding phytol (Daniel Steinberg et al., (1966), J. Lipid Res., vol. 7, pp 684 - 690; Effects of dietary phytol and phytanic acid in animals).

Polyunsaturated fatty acids [that are preferably rumenic acid and conjugated linoleic acids (CLA): the positional isomers of CLA include Δ-7, Δ-9; Δ-8, Δ-10; Δ-9, Δ-11; Δ-10, Δ-12; Δ-11,Δ-13, and Δ-12, Δ-14 conjugated octadecadienoic acids. Each of the aforementioned positional conjugated diene isomers can occur in the following geometric configurations: cis-, trans-; trans-, cis-; cis-, cis- and trans-, trans-. Rumenic acid is the proposed common name of the predominant CLA isomer: cis-9, trans-11); γ-linolenic acid; eicosapentaenoic acid and docosahexaenoic acid] are present in the compositions of the invention in amounts ranging from 0.01 to 500 mg, preferably from 5 to 50 mg per kg body weight of the treated subject.

Vitamins are present in the compositions of the invention in amounts corresponding to their daily dosage ranges used in humans. Particularly preferred ranges are the following:
1) vitamin E : 0.01 to 20 mg, preferably 0.2 to 0.8 mg per kg body weight;
2) lipoic acid: 0.01 to 10 mg, preferably 0.2 to 0.8 mg per kg body weight;
3) vitamin C: 0.1 to 50 mg, preferably 0.5 to 3.0 mg per kg body weight;
4) vitamin B6: 1 to 300 µg, preferably 5 to 50 µg per kg body weight;
5) folates: 0.1 to 30 µg, preferably 2 to 6 µg per kg body weight; and
6) vitamin B 12: 0.001 to 0.3 µg, preferably 0.02 to 0.06 µg per kg body weight.

Terpenes are present in amounts ranging from 0.01 to 100 mg, preferably from 0.5 and 10 mg per kg body weight.

1-Piperoylpiperidine is present in amounts ranging from 0.001 to 5 mg, preferably from 0.02 and 0.2 mg per kg body weight.

Capsaicin is present in amounts ranging from 0.01 to 100 mg, preferably from 0.1 and 7.5 mg per kg body weight.

Polycosanols and/or polycosanolic acids are present in amounts ranging from 0.01 to 50 mg, preferably from 0.1 to 2 mg per kg body weight.

The compositions of the invention can further contain other conventional components, such as oligoelements, mineral salts, bioflavones, extracts of medicinal herbs, phytosterols, amino acids, peptides, etc.

The present invention also relates to the procedures for the preparation of the mixtures of one or more active principles either in the form of oily solutions (see examples 1-3), water-dispersible lipoprotein powders (see examples 4 and 5) or of powders water-dispersible liposaccharide powders (see example 6), as well as to the various oral formulations and functional foodstuffs containing the above mentioned active principles. Oily solutions may be used both for the preparation of suitable functional foodstuffs (dressing oils and oily food derivatives such as margarine, butter, mayonnaise, sauces, creams, chocolate, etc.) and for the galenic formulations consisting of hard- or soft- gelatin capsules containing them.

The water-dispersible lipoprotein powders are prepared by complexing lipophilic active principles with suitable water-soluble protein excipients such as albumins, delipidized albumins, soy-bean proteins, wheat proteins, caseins, milk serum proteins, gelatins, and the like. The water-dispersible liposaccharidic, powders are prepared by complexing the lipophilic active principles with suitable saccharide excipients such as lactose, starches, cellulose, honey or fractions thereof, chitins and chitosans, pectins, inulins, natural fibers, cyclodextrins, and the like.

Both powders can be used for the preparation of a variety of functional foodstuffs, for example by:
a) mixing with flours for the preparation of bread, pasta, cracker, biscuits of other bakery products;
b) addition to fruit juices, soft drinks or other beverages;
c) addition to milk and derivatives thereof (yogurt, puddings, ricotta, cheese, etc.).

Furthermore, said powders can be used for galenic formulations, such as tablets, sugar-coated pills, sachets, effervescent tablets, chewing gums, etc.

Some examples of procedures for the preparation of both the oily mixtures and the powders of the active principles, as well as of the different possible formulations of the invention, are reported in the following:

### Example 1: oily solution of:

a) 18.3 g of a mixture consisting of 60% of fish oil
   (containing 28% of EPA and 18% of DHA),
   20% of borage oil (containing 18% of γ-linolenic acid)
   and 20% of CLA-enriched maize oil (40%) added with
   vitamin E (200 mg), vitamin C palmitate (400 mg),
   lipoic acid (100 mg) g 19
   +
b) terpene fractions of essential oils of lemon (70%),
   thyme (25%), clove (2.5%), and nutmeg (2.5%),
   preferably containing d-limonene, sabinene,
   α-pinene, β-pinene, eugenol, thymol, β-caryophyllene g 0.475
   +
c) 1-piperoylpiperidine mg 100
   +
d) policosanols (80%) and polycosanolic acids (20%)
   from wax rice g 0.425

Mixture a) is heated at 45°-50°C under mild stirring, then slowly added with components b), c) and d) which homogeneously dissolve in a few minutes. The resulting homogeneous oily solution is then cooled at room temperature and stored for further galenic or alimentary uses.

### Example 2: oily solution of:

a) 18.3 g of cod oil containing 18% of EPA
   and 12% of DHA added with vitamin E
   (200 mg), vitamin C palmitate (400 mg),
   lipoic acid (100 mg) g 19
   +
b) terpene fractions of essential oils of lemon (70%),
   nutmeg (10%), thyme (20%) containing
   d-limonene, sabinene, α-pinene, β-pinene, eugenol,
   thymol and β-caryophyllene g 0.475
   +
c) 1-piperoylpiperidine mg 25
   +
d) polycosanols and/or polycosanolic acids from rice wax g 0.5

The oily mixture of the various active principles is prepared as described in Example 1.

### Example 3: oily solution of:

a) 95.9 g of EPA ethyl ester (50%) + DHA ethyl ester (30%)
   + γ-linolenic acid ethyl ester (20%) added with
   vitamin E (600 mg) vitamin C palmitate (1.2 g) and
   lipoic acid (300 mg) g 98
   +
b) terpene fractions of essential oils as in Example 1 g 1
   +
c) 1-piperoylpiperidine as in Example 1 mg 50
d) polycosanols and/or polycosanolic acids from sugar cane
   wax g 0.9

The oily mixture of the various active principles is prepared as described in Example 1.

### Example 4: water-dispersible lipoprotein powders of:

a) delipidized egg albumin or milk caseins or
   soy proteins or a mixture thereof) g 500
   +
b) terpene fractions of essential oils as
   in Example 1 g 5
   +
c) 1-piperoylpiperidine g 0.05
   +
d) polycosanols and/or polycosanolic acids from citrus wax g 5
   +
e) vitaminized mixture of fish oil, oil of borage and CLA
   as in Example 1 g 30

All the components are dissolved under stirring in 2.5 liters of water and mixed for 2/4 minutes in a mechanical blade homogenizer at 20°-25°C, the solution is subsequently spray-dried to a powder, which is the stabilized water-dispersible lipoprotein complex of the lipophilic and vitaminic active principles and can be used as such for the preparation of both pharmaceutical or dietary formulations and various functional foodstuffs.

### Example 5: water-dispersible lipoprotein powders of

a) soy proteins g 250
   +
b) 246.95 g of soy lecithin (rich in linoleic and linolenic
   acids) added with vitamin E(1g), vitamin C
   (2 g), vitamin B6 (50 mg), folic acid (100 µg)
   and vitamin B 12 (100) g 250
   +
c) polycosanols and/or polycosanolic acids from rice wax g 2,5
   +
d) terpene fractions of essential oils as
   in Example 1 g 5
   +
e) 1-piperoylpiperidine g 0.05

The powders are prepared as described in Example 5.

### Example 6: water-dispersible liposaccharidic powders:

a) wheat starch or β-cyclodextrins or pectins or
   cellulose and vegetable fibers of other species) g 200
   +
b) polycosanols and polycosanolic acids from wheat waxes g 1.0
   +
c) terpene fractions of essential oils as in Example 1 g 1.0
   +
d) 1-piperoylpiperidine mg 10
   +
e) 49.150 g of a mixture of linseed oil (50%) and
   borage oil (50%) added with vitamin E
   (100 mg) vitamin C palmitate (200 mg), lipoic acid
   (50 mg) and vitamin B6 (5 mg) g 20

The mixture of the different active principles is obtained as described in Example 4.

### Pharmacological and/or dietetic tests

The pharmacological and/or dietetic characteristics of the compositions of the present invention were experimentally tested on rat and clinically tested on man.

Rats received hypercaloric, hypertriglyceridemizing and hypercholesterolemizing diet. After twenty days of treatment, the effect of the compositions on the following parameters was evaluated:
1) lipoperoxides levels in plasma, liver, brain and heart;
2) body weight;
3) fluidity of the membranes of erythrocyte ghosts and plasma platelets;
4) total cholesterol and HDL cholesterol plasma levels;
5) total triglycerids plasma levels.

80 Male rats, each weighing 150-200 g, were used. The animals were subdivided into 8 groups of 10 animals each:
**1^{st} group:** control (C); 10 animals (control at time 0) received no treatment, 10 animals received for 20 days standard hypercaloric, hyperlipidemizing and hypercholesterolemizing diet consisting of:
   20% casein; 3.5% mixture of oligoelements and mineral salts; 0.1% mixture of vitamins; 0.2% choline ditartrate; 2% cellulose; cholesterol 0.5%; 0.25% sodium cholate; 58.44% saccharose; 10.0% lard and 4.9% olive oil.
**2^{nd} group:** treated with vitaminized fish oil (OP); animals received for 20 days the same diet as the controls, except that 1.9 g of vitaminized fish oil as reported in Example 2 replaced in part a similar amount olive oil (olive oil used: 3.0%).
**3^{rd} group A:** animals were treated with a polycosanols mixture (octacosanol = 50%) of rice wax (PLC), animals received for 20 days the same diet as the controls, except that 0.5 g of rice wax polycosanols replaced in part a similar amount of olive oil (olive oil used: 4.4%).
**3^{rd} group B:** animals were treated with a mixture of polycosanolic acids (PLC ac.) (montanic ≅ 50%) obtained by oxidation of the mixture of rice wax polycosanols of 3^{rd} group A; animals received for 20 days the same diet as the controls, except that 0.5 g of rice wax polycosanolic acids replaced in part a similar amount of olive oil (olive oil used: 4.4%).
**4^{th} group:** animals were treated with a terpene mixture (TRP); animals received for 20 days the same diet as the controls, except that 0.475 g of a terpene mixture as reported in Example 1 replaced in part a similar amount of olive oil (olive oil used: 4.425%).
**5^{th} group**: treated with 1-piperoylpiperidine (P); animals received for 20 days the same diet as the controls, except that 0.025 g of 1-piperoylpiperidine replaced a similar amount of olive oil (olive oil used: 4.875%).
**6^{th} group**: treated with a terpene mixture + 1-piperoylpiperidine + vitaminized fish oil (TRP + P + OP); animals received for 20 days the same diet as the controls, except that 1.9 g of vitaminized fish oil + 0.475 g of a terpene mixture + 0.025 g of 1-piperoylpiperidine replaced a similar amount of olive oil (olive oil used: 2.5%).
**7 ^{th} group:** treated with vitaminized fish oil + a mixture of terpenes + 1-piperoylpiperidine + a mixture of polycosanols (50%) and polycosanolic acids (50%) (OP + TRP + P + PCL); animals received for 20 days the same diet as the controls, except that 1.9 g of vitaminized fish oil, 0.475 g of a terpene mixture, 0.025 g of 1-piperoylpiperidine and 0.5 g of polycosanols and polycosanolic acids replaced 2.9 g of olive oil (olive oil used: 2.0 g).

Results are reported in the following tables I, II and III.

**Table I - Percent changes of the fluidity of the membrane of erythrocyte ghosts and plasma platelets (expressed as % compared with controls values at time 0) of the rats before and after 20 days of dietary treatment.**

| | **membrane fluidity** (erythrocyte ghosts)) | **membrane fluidity** (plasma platelets) |
|---|---|---|
| 1^{st} A control rats at time 0 | 100% | 100% |
| 1^{st} B control rats after 20 1^{st} B control after 20 day diet | 72% | 69% |
| 2^{nd} treated rats (OP) | 76% | 74% |
| 3^{rd} A treated rats (PLC) | 74% | 71% |
| 3^{rd} B treated rats (Ac. PLC) | 82% | 81% |
| 4^{th} treated rats (TRP) | 76% | 71 % |
| 5^{th} treated rats (P) | 73% | 70% |
| 6^{th} treated rats (OP+TRP+P) | 90% | 91% |
| 7^{th} treated rats (OP+TRP+P+PLC) | 96% | 96% |

**Table II - Lipoperoxides levels [expressed as n mols of malonyldialdehyde (MDA) per gram of tissue or per ml of plasma] in plasma, liver, brain and heart of rats before and after 20 day dietary treatment.**

| | MDA PLASMA | Percent change vs. controls | MDA LIVER | Percent change vs. controls | MDA BRAIN | Percent change vs. controls | MDA HEART | Percent change compared with controls |
|---|---|---|---|---|---|---|---|---|
| 1^{st} A control rats at time 0 | 2.5 ± 0.5 | // | 25.5 ± 5.9 | // | 55 ± 4 | // | 24 ± 5 | // |
| 1^{st} B control rats after 20 days of diet | 5.1 ± 0.6 | 100% | 44.2 ± 8.2 | 100% | 108 ± 6 | 100% | 45 ± 6 | 100% |
| 2^{nd} treated rats (OP) | 5.0 ± 0.6 | -1.9% | 45.1 ± 8.2 | 2% | 106 ± 7 | -1.8% | 44 ± 9 | -2.2% |
| 3^{rd} A treated rats (PLC) | 4.8 ± 0.5 | -5.8% | 43.1 ± 6.5 | -2.4% | 105 ± 9 | -2.8% | 42 ± 7 | -6.6% |
| 3^{rd} B treated rats (Ac. PLC) | 3.9 ± 0.5 | -22.0% | 36.2 ± 6.1 | -22.1% | 95 ± 7 | -12.0% | 39 ± 7 | -13.3% |
| 4^{th} treated rats (TRP) | 4.7 ± 0.4 | -7.8% | 41.8 ± 8.2 | -5.4% | 108 ± 7 | -0.0% | 44 ± 9 | -2.2% |
| 5^{th} treated rats (P) | 5.0 ± 0.3 | -1.9% | 43.5 ± 7.8 | -1.5% | 107 ± 5 | -0.9% | 44 ± 7 | -2.2% |
| 6^{th} treated rats (OP+TRP+P) | 3.6 ± 0.4 | -29.4% | 34.7 ± 6.8 | -21.4% | 81 ± 8 | -25.0% | 36 ± 8 | -20% |
| 7^{th} treated rats (OP±TRP+P+PLC) | 2.9 ± 0.4 | -43.1% | 29.9 ± 7.1 | -32.3% | 76 ± 10 | -29.6% | 32 ± 8 | -28.8% |

**Table III - Change of the body weight and of total cholesterol, HDL cholesterol and triglycerids plasma levels in rats before and after 20 days of diet treatments.**

| | Total cholesterol (mg dl⁻¹) | HDL cholesterol (mg dl⁻¹) | total triglycerids (mg dl⁻¹) | body weight (g) |
|---|---|---|---|---|
| 1^{st} A) control rats at time 0 | 35.6 ± 1.8 | 26.2 ± 1.4 | 50.2 ± 7.7 | 180 ± 12 |
| 1^{st} B) control rats after 20 day diet | 126.2 ± 13.5 (100%) | 29.4 ± 1.6 | 82.5 ± 9.5 (100%) | 224 ± 19 (100%) |
| 2^{nd} treated rats (OP) | 120.4 ± 12.7 (-4.5%) | 28.9 ± 2.8 | 80.5 ±6.8 (-2.4%) | 221 ± 16 (-1.3%) |
| 3^{rd} A) treated rats (PLC) | 114.3 ± 10.1 (-12.5%) | 31.6 ± 3.9 | 81.4 ± 8.7 (-1.3%) | 219 ± 18 (-2.2%) |
| 3^{rd} B) treated rats (Ac. PLC) | 89.2 ± 8.1 (-29.3%) | 32.0 ± 4.1 | 75.2 ± 8.5 (-8.8%) | 208 ± 16 (-7.1%) |
| 4^{th}) treated rats (TRP) | 113.9 ± 12.4 (-9.7%) | 29.9±2.0 | 80.4 ± 10.5 (-2.5%) | 219 ± 14 (-2.2%) |
| 5^{th}) treated (P) rats | 125.2 ± 12.5 (-0.7%) | 29.3 ± 1.5 | 81.9 ± 8.7 (-0.7%) | 223 ± 16 (-0.4%) |
| 6^{th}) treated rats (OP+TRP+P) | 71.7 ± 8.9 (-43.1%) | 32.1 ± 3.8 | 72.5 ± 7.9 (-12.1%) | 20) ± 20 (-10.7%) |
| 7^{th}) treated rats (OP+TRP+P+PLC) | 54.8 ± 3.9 (-56.5%) | 32.4 ± 4.1 | 68.7 ± 5.4 (-16.7%) | 186 ± 15 (-16.9%) |

Membrane fluidity of erythrocyte ghosts and plasma platelets was measured using TMA-DPH as fluorescent probe, according to the method by Caimi F. et al., 1999, Thromb. Hoemost., 82 pp. 149.

Malonyldialdehyde was evaluated according to the procedure by K. Yagi et al., 1982, in "Lipid Peroxides in Biology and Medicine", Academic Press, New York, pp. 324-340.

The data reported in tables I, II and III evidence that the compositions of yhe invention are able of promoting, in rats receiving for 20 days a hypercaloric diet and enriched in saturated animal fats and cholesterol, a surprising synergistic effect in:
- restoring membrane fluidity of ghosts and platelets;
- improving antioxidant defenses of plasma, liver, brain and heart;
- reducing excessive increase in body weight;
- limiting excessive increase in plasma cholesterol and triglycerid levels.

Said beneficial and therapeutical effects are always significantly higher than the sum of the beneficial effects obtainable by administering separately the single components of the mixture.

The data reported in Tables I, II and III show that mixtures of polycosanolic acids administered alone always promote, in rats receiving for 20 days a hypercaloric diet enriched in saturated animal fats and cholesterol, a surprisingly higher effect than that obtained with the corresponding mixtures of polycosanols in:
- restoring membrane fluidity of ghosts and platelets;
- improving antioxidant defenses of plasma, liver, brain and heart;
- reducing excessive increase in body weight;
- limiting excessive increase in plasma cholesterol and triglycerid levels.

Clinical trials were carried out on different groups of patients both healthy and suffering from one or more of the many aging-related dysmetabolic disorders (atherosclerosis; obesity and overweight; hypercholesterolemia and/or hypertriglyceridemia; hypertension; diabetes; cerebral-degenerative diseases such as Alzheimer, Parkinson, senile dementia, loss of memory and the like; stress and/or depression; menopausal syndromes; prostate hypertrophy; osteoporosis; aging of the skin; panniculopathies (cellulite); alopecia). Patients were subjected to dietotherapic treatment with the mixtures reported in Example 1 or in Example 2. Doses and times varied depending on the treated groups.

In all clinical trials, the beneficial therapeutical effects obtainable with the administration of the mixtures reported in Example 1 or in Example 2 were always significantly high, in the prevention of aging biological symptoms (better membrane fluidity, improvement of antioxidant defenses, limitation of weight excesses) and in the improvement of some of the tested clinical parameters; therefore said compositions may be usefully employed both is aging prevention and as coadjuvants and synergetic agents to improve the therapeutical action of drugs already normally in use in the treatment of the above cited diseases.

Furthermore, said beneficial aspects were always by far higher than the sum of the effects obtainable by the administrations of the single active principles of the different mixtures.

Finally, these clinical trials in humans showed that the administration of a mixture of polycosanolic acids alone was surprisingly more effective in preventing aging biochemical symptoms (better membrane fluidity, improvement of antioxidant defenses, limitation of weight excesses, reduction of plasma cholesterol and triglycerides, etc.) than the administration of the mixture of the corresponding polycosanols alone.

## Claims

1. Pharmaceutical, dietetic or alimentary compositions containing:
a) a lipidic mixture rich in polyunsaturated acids and vitamins, in combination with;
b) one or more terpenes selected from monoterpenes and/or sesquiterpenes;
c) 1-piperoylpiperidine in the pure form and/or extracts or purified fractions enriched in black pepper containing it and optionally
d) one or more polycosanols and/or polycosanolic acids.

2. Compositions as claimed in claim 1, wherein monoterpenes are selected from d-limonene, β-caryophyllene, sabinene, borneol, carveol, carvone, eugenol, eugenol acetate, geraniol, menthol, myrtanol, pinene, β-pinene, thymol.

3. Compositions as claimed in claim 1, wherein sesquiterpenes are selected from β-ionone, rodinal, abscisic acid.

4. Compositions as claimed in claim 1, wherein polycosanols are selected from octacosanol, triacosanol, tetracosanol, hexacosanol.

5. Compositions as claimed in claim 1, wherein the polycosanolic acid is montanic acid.

6. Compositions as claimed in claim 1, wherein the lipidic mixture rich in unsaturated acids consists of an oil selected from fish, maize, soy-bean, sunflower, wheat germ, borage, red currant, evening primrose, olive oils.

7. Compositions as claimed in any one of the above claims further containing mineral salts, oligoelements, amino acids, phytosterols, bioflavones, plants extracts.

8. Compositions as claimed in claims 1-7 as agents capable of limiting the increase in body weight and membrane stiffness, having hypocholesterolemizing, hypogliyeridemizing and antioxidant activities.

9. Water-dispersible lipoprotein powders consisting of components a), b), c) and d) as defined in claim 1 and a water-soluble protein.

10. Lipoprotein powders as claimed in claim 9, wherein the water-soluble protein is selected from albumin, egg albumin, delipidized albumin, soy proteins, caseins, gelatins.

11. A process for the preparation of the lipoprotein powders of claims 9 and 10, which comprises subjecting to spray-drying a solution of components a), b), c) and d) and a water-soluble protein.

12. Water-dispersible liposaccharide powders consisting of components a), b), c) and d) as defined in claim 1 and of a di- or polysaccharide.

13. Liposaccharide powders as claimed in claim 12, wherein the di- or polysaccharide is selected from saccharose, lactose, starches, cellulose, chitins, chitosans, pectins, insulin, cellulose fibers, cyclodextrins, honey or fractions thereof.

14. A process for the preparation of the liposaccharide powders of claims 12, and 13, which comprises subjecting to spray-drying an aqueous solution of components a), b), c) and d) and di- or polysaccharide.

15. The use of the lipoprotein powders of claims 9 and 10 and of the liposaccharide powders of claims 12 and 13 for the preparation of foodstuffs and galenic formulations.

16. Functional foodstuffs containing the water-dispersible powders of claims 9-10 and 12-13.

## Patentansprüche

1. Pharmazeutische, diätetische oder zur Nahrung dienende Zusammensetzungen, beinhaltend
a) eine lipidische Mischung, die reich ist an mehrfach ungesättigten Säuren und Vitaminen, in Kombination mit:
b) einem oder mehr aus Monoterpenen und/oder Sesquiterpenen ausgewählten Terpenen;
c) 1-Piperoylpiperidin in der reinen Form und/oder mit schwarzem Pfeffer angereicherte Extrakte oder gereinigte Fraktionen, die es enthalten, und optional
d) eines oder mehr Polycosanole und/oder Säuren von Polycosanolen.

2. Zusammensetzungen nach Anspruch 1, wobei Monoterpene aus d-Limonen, β-Caryophyllen, Sabinen, Borneol, Carveol, Carvon, Eugenol, Eugenolacetat, Geraniol, Menthol, Myrtanol, Pinen, β-Pinen, Thymol ausgewählt werden.

3. Zusammensetzungen nach Anspruch 1 , wobei Sesquiterpene aus β-lonon, Rodinal, Abscisinsäure ausgewählt werden.

4. Zusammensetzungen nach Anspruch 1, wobei die Polycosanole aus Octacosanol, Triacosanol, Tetracosanol, Hexacosanol ausgewählt werden.

5. Zusammensetzungen nach Anspruch 1, wobei die Säuren von Polycosanolen Montansäure ist,

6. Zusammensetzungen nach Anspruch 1, wobei die an mehrfach ungesättigten Säuren reiche lipidische Mischung aus einem Öl besteht, das aus Fisch-, Mais-, Sojabohnen-, Sonnenblumen-, Weizenkeim-, Borretsch-, Johannisbeer-, Nachtkerzen-, Olivenölen ausgewählt wird.

7. Zusammensetzungen nach irgendeinem der obigen Ansprüche, des weiteren beinhaltend Mineralsalze, Oligoelemente, Aminosäuren, Phytosterole, Bioflavone, Pflanzenextrakte.

8. Zusammensetzungen nach den Ansprüchen 1 - 7, als Wirkstoffe, die in der Lage sind, den Zuwachs an Körpergewicht und Membransteifigkeit zu begrenzen, die cholesterinsenkende, glyceridsenkende und antioxidante Aktivitäten aufweisen.

9. Wasser-dispergierbare Lipoproteinpulver, die aus den wie in Anspruch 1 definierten Komponenten a), b), c) und d) und einem wasserlöslichen Protein bestehen.

10. Lipoproteinpulver nach Anspruch 9, wobei das wasserlösliche Protein aus Albumin, Eialbumin, delipidiertem Albumin, Sojaproteinen, Caseinen, Gelatinen ausgewählt wird.

11. Ein Verfahren zur Herstellung der Lipoproteinpulver nach den Ansprüchen 9 und 10, das umfasst, eine Lösung der Komponenten a), b), c) und d) und eines wasserlöslichen Proteins der Trocknung im Sprühverfahren zu unterziehen.

12. Wasser-dispergierbare Liposaccharidpulver, die aus den wie in Anspruch 1 definierten Komponenten a), b), c) und d) und einem Di-oder Polysaccharid bestehen.

13. Liposaccharidpulver nach Anspruch 12, wobei das Di- oder Polysaccharid aus Saccharose, Lactose, Stärken, Cellulose, Chitinen, Chitosanen, Pektinen, Insulin, Cellulosefasern, Cyclodextrinen, Honig oder Fraktionen davon ausgewählt wird.

14. Ein Verfahren zur Herstellung der Liposaccharidpulver nach den Ansprüchen 12 und 13, das umfasst, eine wässerige Lösung der Komponenten a), b), c) und d) und eines Di- oder Polysaccharid der Trocknung im Sprühverfahren zu unterziehen.

15. Die Verwendung der Lipoproteinpulver der Ansprüche 9 und 10 und der Liposaccharidpulver der Ansprüche 12 und 13 zur Herstellung von Nahrungsmitteln und galenischen Formulierungen.

16. Funktionelle Nahrungsmittel, beinhaltend die wasser-dispergierbaren Pulver der Ansprüche 9 - 10 und 12 - 13.

## Revendications

1. Compositions pharmaceutiques, diététiques ou alimentaires contenant :
a) un mélange lipidique riche en acides polyinsaturés et en vitamines, en combinaison avec :
b) un ou plusieurs terpènes choisis parmi des monoterpènes et/ou des sesquiterpènes ;
c) une 1-pipéroylpipéridine sous la forme pure et/ou des extraits ou des fractions purifiées enrichi(e)s en poivre noir contenant ladite 1-pipéroylpipéridine et éventuellement
d) un ou plusieurs polycosanols et/ou acides polycosanoliques

2. Compositions selon la revendication 1, dans lesquelles les monoterpènes sont choisis parmi le d-limonène, le β-caryophyllène, le sabinène, le bornéol, le carvéol, la carvone, l'eugénol, l'acétate d'eugénol, le géraniol, le menthol, le myrtanol, le pinène, le β-pinène, le thymol.

3. Compositions selon la revendication 1, dans lesquelles les sesquiterpènes sont choisis parmi la β-ionone, le rodinal, l'acide abscisique.

4. Compositions selon la revendication 1, dans lesquelles les polycosanols sont choisis parmi l'octacosanol, le triacosanol, le tétracosanol, l'hexacosanol.

5. Compositions selon la revendication 1, dans lesquelles l'acide polycosanolique est un acide montanique.

6. Compositions selon la revendication 1, dans lesquelles le mélange lipidique riche en acides insaturés est constitué d'une huile choisie parmi les huiles de poisson, de maïs, de soja, de tournesol, de germe de blé, de bourrache, de groseille rouge à grappes, d'onagre, d'olive.

7. Compositions selon l'une quelconque des revendications ci-dessus, contenant en outre des sels minéraux, des oligoéléments, des acides aminés, des phytostérols, des bioflavones, des extraits de plantes.

8. Compositions selon les revendications 1 à 7, en tant qu'agents capables de limiter l'augmentation du poids corporel et de la raideur membranaire, ayant des activités hypocholestérolémique, hypogliyéridémique et antioxydante.

9. Poudres de lipoprotéine hydrodispersables constituées des composants a), b), c) et d) tels que définis dans la revendication 1 et d'une protéine hydrosoluble.

10. Poudres de lipoprotéine selon la revendication 9, dans lesquelles la protéine hydrosoluble est choisie parmi une albumine, une ovalbumine, une albumine délipidée, des protéines de soja, des caséines, des gélatines.

11. Procédé de préparation des poudres de lipoprotéine selon les revendications 9 et 10, qui comprend la soumission d'une solution de composants a), b), c) et d) et d'une protéine hydrosoluble à un séchage par pulvérisation.

12. Poudres de liposaccharide hydrodispersables constituées des composants a), b), c) et d) tels que définis dans la revendication 1 et d'un di- ou polysaccharide.

13. Poudres de liposaccharide selon la revendication 12, dans lesquelles le di- ou polysaccharide est choisi parmi le saccharose, le lactose, les amidons, la cellulose, les chitines, les chitosans, les pectines, l'insuline, les fibres de cellulose, les cyclodextrines, le miel ou leurs fractions.

14. Procédé de préparation des poudres de liposaccharide selon les revendications 12 et 13, qui comprend la soumission d'une solution aqueuse de composants a), b), c) et d) et de di- ou polysaccharide à un séchage par pulvérisation.

15. Utilisation des poudres de lipoprotéine selon les revendications 9 et 10 et des poudres de liposaccharide selon les revendications 12 et 13 pour la préparation de produits alimentaires et de formulations galéniques.

16. Produits alimentaires fonctionnels contenant les poudres hydrodispersables selon les revendications 9-10 et 12-13.
